(19) Europäisches Patentamt

European Patent Office

Office européen des brevets

(11) EP 0 678 022 B1

## (12) FASCICULE DE BREVET EUROPEEN

(45) Date de publication et mention
de la délivrance du brevet:
**13.11.1996 Bulletin 1996/46**

(21) Numéro de dépôt: **94903934.1**

(22) Date de dépôt: **03.01.1994**

(51) Int. Cl.$^6$: **A61K 31/425**

(86) Numéro de dépôt international:
**PCT/FR94/00002**

(87) Numéro de publication internationale:
**WO 94/15600 (21.07.1994 Gazette 1994/17)**

(54) **APPLICATION DU RILUZOLE COMME RADIORESTAURATEUR**

VERWENDUNG VON RILUZOL ZUM SCHUTZ GEGEN STRAHLENSCHÄDEN

APPLICATION OF RILUZOLE AS A RADIORESTORER

(84) Etats contractants désignés:
**AT BE CH DE DK ES FR GB GR IE IT LI LU NL PT SE**

(30) Priorité: **07.01.1993 FR 9300073**

(43) Date de publication de la demande:
**25.10.1995 Bulletin 1995/43**

(73) Titulaire: **RHONE-POULENC RORER S.A.**
**92160 Antony (FR)**

(72) Inventeurs:
• **PRATT, Jérémy**
**F-94220 Charenton-le-Pont (FR)**
• **STUTZMANN, Jean-Marie**
**F-94440 Villecresnes (FR)**

(74) Mandataire: **Savina, Jacques et al**
**Rhône-Poulenc Rorer S.A.**
**Direction Brevets (t144)**
**20 avenue Raymond Aron**
**92165 Antony Cédex (FR)**

(56) Documents cités:
**EP-A- 0 050 551    EP-A- 0 282 971**
**WO-A-91/17984**

• **J. NEUROSCI. vol. 9, no. 11 , 1989 pages 3720 - 3727 C. MALGOURIS ET AL. 'Riluzole. a novel antiglutamate, prevents memory loss and hippocampal neuronal damage in ischemic gerbils'**
• **DATABASE WPI Week 8511, Derwent Publications Ltd., London, GB; AN 85-067485 & SU,A,1 109 392 (AS LITH CHEM ENG IN) 23 Aoüt 1984**
• **DATABASE WPI Week 8511, Derwent Publications Ltd., London, GB; AN 85-067485; & SU-A-1 109 392**

**Description**

La présente invention concerne une nouvelle application thérapeutique du riluzole (trifluorométhoxy-6 amino-2 benzothiazole) ou les sels pharmaceutiquement acceptables de ce composé.

Le riluzole est utile comme médicament anticonvulsivant, anxiolytique et hypnotique (brevet EP 50551), dans le traitement de la schizophrénie (EP 305276), dans le traitement des troubles du sommeil et de la dépression (EP 305277), dans le traitement des désordres cérébrovasculaires et comme anesthésique (EP 282971).

Il a maintenant été trouvé de manière surprenante que ce composé peut aussi être utilisé comme radiorestaurateur.

La radiorestauration est utile dans la thérapie aux rayons X notamment dans le traitement des cancers et contre les autres sources de radiations nuisibles telles que celles rencontrées par les personnes dans les zones limitrophes des explosions nucléaires.

L'activité du produit a été mise en évidence sur le rhinencéphale de jeune rat soumis à une irradiation gamma globale.

L'irradiation s'effectue au moyen d'une source de rayons gamma, Cobalt 60.

Les animaux utilisés sont des rats mâles de souche Sprague Dawley pesant 28 à 33 g agés de 15 jours qui sont placés dans une boite de contention aérée en plexiglass subissant une rotation de 180° afin de réaliser des irradiations globales et homogènes en dose unique de 1,5 et 2,5 Gy dont le débit de dose est de 0,2 Gy par minute. Le délai de survie entre l'irradiation et le sacrifice est de 6 heures. Tous les animaux sont fixés par perfusion intra-aortique d'un liquide fixateur composé de 1% de paraformaldéhyde, 1% de glutaraldéhyde et 0,05% de chlorure de calcium dans un tampon phosphate 0,4M de pH 7,3. Pour éviter la coagulation, on injecte 0,04 ml d'héparine dans le ventricule et 0,3 ml de nitrite de sodium à 1% pour débarasser les vaisseaux des hématies.

Les animaux sont anesthésiés par injection intrapéritonéale de Pentobarbital sodique à 3%. Les animaux sont ensuite couchés sur le dos et fixés sur la table d'opération. La cage thoracique est ouverte et maintenue béante à l'aide de 2 clamps. Le coeur est ainsi dégagé, la pointe du ventricule gauche est incisée, la canule de perfusion introduite jusqu'au début de la crosse de l'aorte et clampée. L'oreillette droite est incisée et la perfusion est effectuée. Le passage du liquide de perfusion est assuré par gravité. Après la perfusion, la tête de l'animal est coupée et le cerveau est prélevé, immergé dans le liquide fixateur et conservé une nuit à 4°C.

Le lendemain de la perfusion, on procède à des coupes frontales du gyrus dentatus sous une loupe binoculaire. Les fragments recueillis sont immergés dans le liquide de lavage pendant 5 minutes. Ils sont ensuite déshydratés dans des bains d'alcool de concentration croissante puis inclus dans l'araldite. Les coupes semi-fines de 1 micromètre sont réalisées à l'aide d'un Ultramicrotome Reichert avec des couteaux de verre. Elles sont colorées à chaud avec une solution filtrée de bleu de toluidine à 1% préparée dans du tampon borate à 1% puis observées à l'aide d'un microscope Orthoplan.

L'étude comparative consiste à compter sur 3 coupes non sériées (séparées de 10 micromètre chacune), pour chaque rat et sur un ensemble de 1000 cellules au total (granulaires et subgranulaires). On comptabilise le nombre de cellules pycnotiques puis le nombre de cellules survivantes observées dans cette zone. Ceci permet de calculer le pourcentage de cellules survivantes par rapport au nombre de cellules de la zone ( pourcentage de survie = 100 x cellules vivantes / cellules vivantes + cellules pycnotiques ).

Le produit à étudier est administré par voie intrapéritonéale aux doses de 1, 2, 4 et 8 mg/kg 20 minutes après l'irradiation (au début de la pycnose).

Les résultats obtenus sont mentionnés dans les tableaux suivants et montrent que, après traitement avec le produit testé, la dégénérescence neuronale est inférieure par rapport aux témoins irradiés ne recevant pas de produit.

| ESSAI 1 | | |
|---|---|---|
| IRRADIATION | TEMOINS SURVIE CELLULAIRE | RILUZOLE (2 mg/kg) SURVIE CELLULAIRE |
| 1,5 Gy | 88,8% | 91,2% |
| 2,5 Gy | 87,1% | 92,2% |

| ESSAI 2 | | | | | |
|---|---|---|---|---|---|
| IRRADIATION | TEMOINS SURVIE CELLULAIRE | RILUZOLE 1 mg/kg SURVIE CELLULAIRE | RILUZOLE 2 mg/kg SURVIE CELLULAIRE | RILUZOLE 4 mg/kg SURVIE CELLULAIRE | RILUZOLE 8 mg/kg SURVIE CELLULAIRE |
| 2,5 Gy | 75,35±2,4 % | 81,99±2,32% | 83,54±1,96% | 86,07±2,78 | 85,41±2,14% |

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale ou parentérale.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les doses dépendent de l'effet recherché, de la durée du traitement et de la voie d'administration utilisée; elles sont généralement comprises entre 50 et 800 mg par jour par voie orale pour un adulte avec des doses unitaires allant de 25 à 200 mg de substance active et entre 25 et 600 mg par jour par voie intraveineuse pour un adulte avec des doses unitaires allant de 12,5 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

Exemple A

On prépare, selon la technique habituelle, des comprimés dosés à 50 mg de produit actif ayant la composition suivante :

| | |
|---|---|
| - Riluzole | 50 mg |
| - Mannitol | 64 mg |
| - Cellulose microcristalline | 50 mg |
| - Polyvidone excipient | 12 mg |
| - Carboxyméthylamidon sodique | 16 mg |
| - Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) q.s.p. 1 comprimé pelliculé terminé à 245 mg | |

Exemple B

On prépare, selon la technique habituelle, des gélules dosées à 50 mg de produit actif ayant la composition suivante :

| | |
|---|---|
| - Riluzole | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

Exemple C

On prépare une solution injectable contenant 10 mg de produit actif ayant la composition suivante :

| | |
|---|---|
| - Riluzole | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau | q.s.p. 4 cm3 |

**Revendications**

1.  Application du riluzole ou les sels pharmaceutiquement acceptables de ce composé à la préparation de médicaments utiles comme radiorestaurateurs.

2.  Application selon la revendication 1 pour obtenir un médicament utile par voie orale comprenant 25 à 200 mg du riluzole.

3.  Application selon la revendication 1 pour obtenir un médicament utile par voie intraveineuse comprenant 12,5 à 200 mg du riluzole.

**Claims**

1.  Use of riluzole or the pharmaceutically acceptable salts of this compound in the preparation of medicaments which can be used to promote restoration following irradiation.

2.  Use according to claim 1, for obtaining a medicament which can be used via the oral route comprising 25 to 200 mg of riluzole.

3.  Use according to claim 1, for obtaining a medicament which can be used via the intravenous route comprising 12.5 to 200 mg of riluzole.

**Patentansprüche**

1.  Verwendung von Riluzol oder den pharmazeutisch akzeptablen Salzen dieser Verbindung zur Herstellung von Arzneimitteln für die Behandlung von Strahlenschäden.

2.  Verwendung nach Anspruch 1 zur Herstellung eines auf oralem Wege anwendbaren Arzneimittels, das 25 mg bis 200 mg Riluzol umfaßt.

3.  Verwendung nach Anspruch 1 zur Herstellung eines auf intravenösem Wege anwendbaren Arzneimittels, das 12,5 mg bis 200 mg Riluzol umfaßt.